# EUROPEAN PATENT SPECIFICATION

(11) **EP 0 809 995 B1**
(45) Date of publication and mention of the grant of the patent: **11.09.2002**
(21) Application number: 97106785.5
(22) Date of filing: 24.04.1997
(51) Int. Cl.: A61K 9/16, A61K 31/22

(54) **New diacerhein formulations obtained by active principle inclusion in polysaccharide hydrogels**
Neue Diacerhein Formulierungen, erhalten durch Einschluss des Wirkstoffs in Polysaccharidhydrogele
Nouvelles formulations de la diacerhéine obtenues par inclusion du principe actif dans des hydrogels polysaccharidiques

(30) Priority: 29.04.1996 IT MI960834
(43) Date of publication of application: 03.12.1997
(73) Proprietor: LABORATOIRE MEDIDOM S.A., 1211 Genève 12 (CH)
(72) Inventor: Di Napoli, Guido, 1245 Collonge-Bellerive (CH)
(74) Representative: Gervasi, Gemma, Dr.

(56) References cited:
- EP-A- 0 264 989
- EP-A- 0 446 753
- JOURNAL OF PHARMACEUTICAL SCIENCES, vol. 78, no. 11, November 1989, WASHINGTON (US), pages 964-967, XP000080612 R. BODMEIER ET AL.: "SPHERICAL AGGLOMERATES OF WATER-INSOLUBLE DRUGS"

## Description

### Field of the invention

The present invention relates to new diacerhein-containing formulations for pharmaceutical use and in particular hydrogel matrices based on hydrophilic polysaccharides, such as pectins and alginates, whereinto diacerhein is incorporated, providing a better intestinal tolerability and a higher bioavailability than the pharmaceutical preparations found in commerce.

Diacerhein (DAR), 9,10-dihydro-4,5-diacetoxy-9,10-dioxyanthracene-2-carboxylic acid, is the diacetyl derivative of rhein, an anthraquinone compound present in some varieties of rhubarb (Rheum Chinense). DAR is the active principle of pharmaceutical preparations for oral use, already registered and commercially available in various countries for the treatment of osteoarthrosis.

Unlike other non-steroid drugs, diacerhein exhibits a good gastric tolerability. However, the commercially available formulations of diacerhein, when administered to patients particularly sensitive to anthraquinone derivatives or with enterocolitic troubles, due to high diacerhein doses, or to a combination of the foregoing factors, may have a laxative effect with more or less severe and/or frequent diarrhea episodes.

Alginates are linear polysaccharides composed of units of β-D-mannuronic and α-D-glucuronic acids linked together by (1-4) glycosidic bonds in varying percentage quantities and sequences. The mannuronic acid content ranges from less than 30% to almost 100%, the latter value being found in some alginates of bacterial origin. Also the distribution of the two monomers varies to a considerable extent when passing from rigorously alternate alginates sequences (of bacterial origin) to long homopolymeric sequences of the two monomers present in some samples.

Alginate forms gel by interaction with bivalent ions, such as calcium. It is used in the food and pharmaceutical industries for thickening, suspending and emulsifying and in biotechnology as an encapsulating matrix of microorganisms and/or cells.

Pectins, which are constituents of-the extracellular matrix of plants, consist chiefly of D-galacturonic acid linear chains linked together by (1-4) glycosidic bonds, partially esterified with methanol. The polymer structure regularity is however interrupted by L-ramnose units and by the presence of side chains containing other neutral sugars.

Pectins esterification degree (ED) is expressed as the per cent ratio of esterified galacturonic acid units to the total galacturonic acid units and affects pectins solubility and ability to form gel. When ED is lower than 50%, pectins have a low methoxyl content (LM pectins) and when ED is higher than 50%, pectins have a high methoxyl content (HM pectins).

### State of the art

Said polysaccharides, which for commercial purposes are generally derived from apples and citrus fruit peel, find application in the food industry as fundamental components of jams and jellies, and in the food and pharmaceutical industries as thickeners and suspending agents. Hydrosoluble pectin in combination with other active ingredients, such as yeasts and ferments, or active carbon is administered as an antidiarrheal agent at dosage rates of several grams/day (French patent FR 2996, abstract from Derwent data bank).

European patent application EP-A-648,495 discloses enteral liquid compositions, containing proteins, aminoacids, carbohydrates, fatty acids vitamins and minerals, enriched with water insoluble complexes of anionic polysaccharides, such as pectins and alginates, with cations, such as Ca⁺⁺ ions. These enteral compositions are intended to reduce diarrheal disturbances associated with administration of liquid nutritionals and with surgically shortened bowel.

According to this document, said complexes are administered at dosage rates of at least 5-6 g/day.

None of the prior art documents hint that said polysaccharide complexes may inhibit the diarrhea caused by laxative substances, e.g. sennosides, or by pharmacologically active substances having a laxative side effect, e.g. diacerhein, and/or that they may increase the bioavailability thereof.

Conversely, it is known that resistance of the laxative action of sennosides is decreased by the simultaneous administration of alginates (JP 57145812, abstract from Derwent data bank).

### Brief description of the drawings

Fig. 1 is a graph illustrating the body weight variation in rats treated with commercial ART 50 and with formulations D6, D8 and D9 of the present invention.

Fig. 2 and Fig. 3 show the results of gastrointestinal tolerability tests on rats treated with diacerhein formulations according to the present invention, expressed as the number of rats with at least one diarrhea episode (Fig. 2) and the number of diarrhea episodes recorded in all rats suffering from diarrhea (Fig. 3).

Fig. 4 and Fig. 5 show the results of a bioavailability test conducted on rats after administration of the formulations according to the present invention in amounts corresponding to 40 mg/kg (Fig. 4) and 2 mg/kg (Fig. 5) of diacerhein. The results are expressed as urinary excretion of diacerhein during 24 hours. Each column of the graph corresponds to the result observed per each treated animal.

Fig. 6 shows the calibration curve for the determination of the diacerhein content (evaluated as rhein) in biological fluids.

### Summary

It has surprisingly been found that by combining diacerhein with a gel based on biocompatible gel forming (gelogenic) polysaccharides, in particular by incorporating diacerhein into hydrogel of anionic polysaccharides, such as pectins and alginates partially or totally converted into the corresponding calcium salts, diacerhein formulations so obtained drastically decrease the occurrence and/or severity of diarrhea episodes when compared with the pharmaceutical compositions containing the same active ingredient, such as ART 50 R and Fisiodar ^{R}, without modifying the pharmacological properties of diacerhein and at the same time providing a good bioavailability of the active ingredient.

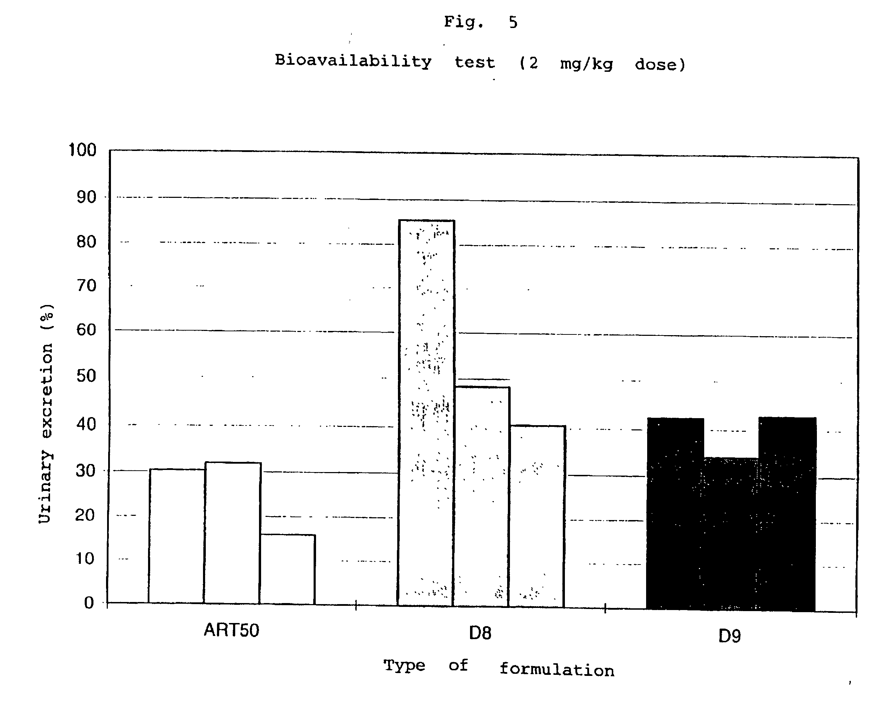

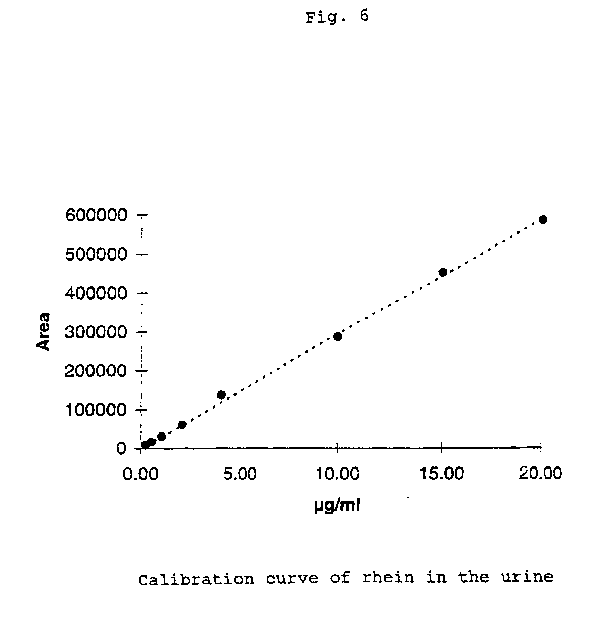

It has also been found that diacerhein incorporation into polysaccharide hydrogels, in particular pectin and alginate hydrogels, yields compositions with an unexpectedly higher diacerhein bioavailability, when compared with commercial compositions, e.g. Fisiodar ^{R} and ART 50 ^{R} (see Figs. 5 and 6).

It is an aspect of the present invention the provision of diacerhein formulations, wherein diacerhein is incorporated into a gel matrix, in particular a hydrogel matrix, including at least an ionic polysaccharide gelled by addition of electrolytes, optionally containing other pharmaceutically acceptable excipients or diluents or other useful ingredients, other than said polysaccharides or mixtures thereof.

It is a further aspect of the present invention the provision of a process for the preparation of the claimed formulations.

According to another aspect of the present invention, the aforesaid formulations, optionally mixed with excipients and/or diluents, are used for the preparation of pharmaceutical compositions, in particular for oral administration, intended in particular for antiinflammatory and analgesic use, more specifically for the treatment of osteoarthrosis.

### Detailed description of the invention

In the claimed formulations, diacerhein is incorporated, i.e. dispersed in a substantially uniform manner, into a polysaccharide gel matrix, in particular a hydrogel (aqueous gel) matrix.

The formulations of the present invention are typically in the form of microgranules, referred to herein also as microspheres or microcapsules, with particle size (particle average diameter) ganerally ranging from 5 to 500 micrometers (µm), preferably from 25 to 350, µm and more preferably from 50 to 100 µm.

The gel of the present invention may be administered as such or, after optional mixing with conventional additives useful for improving its processability, such as lubricants, disgregating agents, fillers, suspending agents, or other useful additional ingredients, may be formulated according to conventional techniques to yield pharmaceutical compositions, in particular for oral administration, such as suspensions, emulsions, capsules, tablets. Excipients and additional ingredients other than gelogenic polysaccharides may be included in the gel matrix or added to the preformed gel microgranules.

The pharmaceutical formulations and compositions according to the present invention are administered in human therapy at dose rates typically ranging from 0.3 to 3.0 mg of diacerhein per kg of body weight per day (mg/kg/day), with diacerhein unit dosages typically of about 25-100 mg.

The polysaccharides suitable for the preparation of the claimed formulations are gelogenic, i.e. gel-forming, and biocompatible, such as for example those reported in pharmacopeias.

The specific gel formation conditions vary depending on the type of polymer.

According to a particularly preferred embodiment, the gel matrix of the formulations according to the present invention comprises one or more anionic polysaccharides, at least partially salified (or complexed) with gel-forming cations.

Therefore, the polysaccharides preferably used for the preparation of the claimed formulations are anionic polysaccharides that gel by the action of cationic reagents and are typically hydrophilic water soluble polysaccharides, either natural or semisynthetic. Particularly preferred are the polysaccharides containing carboxyl groups, such as pectins, alginates and polygalacturonans, and sulphate groups, such as carrageenans.

The anionic polysaccharides useful as starting materials for the preparation of the claimed formulations are preferably in the form of soluble salts, e.g. of alkaline metals (sodium and potassium), such as alginic acid soluble salts (e.g. sodium alginate) and polygalacturonic acid soluble salts (e.g. sodium polygalacturonate).

The use as starting materials of anionic polysaccharides, at least partially acidified (in which the counterion is H⁺) is possible though less practical, the polymers being scarcely soluble. Acid polysaccharides should be preferably neutralized and converted into soluble salts, e.g. sodium or potassium salts, before being mixed with diacerhein and gelled.

The polymer molecular weight may vary within a wide range, provided that its value is suitable for gel formation. For the present scope, the polysaccharides used have a molecular weight preferably ranging from 100,000 daltons to 200,000-300,000 daltons, but polymers having different molecular weights may also be used.

For the purposes of the present invention, particularly preferred are the formulations with a gel matrix derived from polysaccharides with carboxylated groups, such as alginates, pectins and polygalacturonans.

Still more preferred are the formulations containing gels derived from alginates or pectins or mixtures thereof, in particular the formulations with a gel derived from pectins.

The alginates useful for the purposes of the present invention are typically derived from algae. Some alginates of bacterial origin may be used, provided they are selected from those bringing about gel formation by cations addition.

The pectins useful for the purposes of the present invention are particularly of vegetable origin, like those derived from apples and citrus fruit peels.

More preferred are high methylation pectins (ED higher than 50%, preferably from 70% to 95%), in so far as they allow the incorporation of higher amounts of diacerhein, when compared with less methylated pectins, and yield a final product exhibiting more satisfactory technological properties.

For example, it is possible to use a commercial HM pectin having a methylation degree of about 69% and a molecular weight ranging from 100,000 to 150,000 daltons.

The cations useful for complexing anionic polysaccharides causing their gelling are typically cations of alkaline earth metals, e.g. Ca⁺⁺, or other polyvalent cations, e.g. Al⁺⁺⁺, or transition metals cations, particularly bivalent, , e.g. Cu⁺⁺. Particularly preferred are Ca⁺⁺ ions.

According to preferred embodiments of the present formulations, the hydrogel matrix comprises at least an anionic polysaccharide containing carboxylic groups, selected from the group consisting of pectins, alginates and mixtures thereof, at least partially salified (or complexed) with Ca⁺⁺ ions, the hydrogel matrices derived from pectins complexed with Ca⁺⁺ ions being still more preferred.

According to typical embodiments of the present formulations, the gelogenic ionic polysaccharide is the only polymeric constituent of the hydrogel matrix.

According to some embodiments of the present formulations, hydrogels contain the aforesaid anionic polysaccharides, in particular carboxylated, partially salified with gel-forming cations, preferably with Ca⁺⁺ ions, and partially in the H⁺ form.

The gelogenic polymer/diacerhein weight by weight ratio typically ranges between 0.1:1 and 10:1 and preferably between 1:1 and 3:1.

Typical embodiments of the present formulations contain from about 10% to about 50% of diacerhein, and from about 10% to about 80% by weight, and more preferably from 15% to 45% by weight of polysaccharide, typically anionic.

The quantity of water present in the compositions of the present invention may vary within wide limits, being determined by the drying method, and typically ranges from 5% to 20% by weight on the whole composition.

Also the quantity of gel-forming cations may vary within wide limits, being determined, e.g. by the quantity that salifies the anionic groups of gelogenic polysaccharides and by the quantity of inorganic salts, such as e.g. CaCl₂, incorporated into the gel. Typical embodiments of the present invention contain from 1% to 30% by wt. of gel-forming cations, typically Ca⁺⁺, more preferably up to 10% by weight of alkaline metal cations.

Typical embodiments of the present invention contain lactose, which is either incorporated, as an excipient, into the gel matrix or is mixed to preformed gel granules, in an amount ranging, e.g. from 10% to 80% by weight. By weight per cent amounts are referred to the dried formulation total weight.

The formulations according to the present invention are obtained by a process comprising gelling a mixture of diacerhein and the selected polymer in a suitable aqueous diluent, in particular water, separating the gel formed from the excess of the diluent liquid phase, and drying the gel.

Gelling is typically obtained by mixing a polysaccharide with electrolytes and in particular by contacting an anionic polysaccharide with gel-forming cations.

Gelling is preferably conducted on anionic polysaccharides, preferably alginates and pectins, in the form of water soluble salts, in particular of alkaline metal salts, and the cations are preferably Ca⁺⁺ ions, typically added in the form of water soluble salts.

According to a typical embodiment, the claimed process comprises the following steps: pouring under stirring an aqueous suspension containing diacerhein and the selected polymer into a water solution containing gel-forming cations; stirring the resulting mixture for a period typically ranging from about 15 min to about 2 hrs, generally at least 30 min, to stabilize the gel that forms by contact with the water solution containing the aforesaid cations; separating the gel from the aqueous phase, and drying.

According to a typical embodiment of the present invention, the gel is directly obtained in the form of microspheres, herein referred to also as microcapsules, by dropping a homogeneous aqueous suspension containing diacerhein and the gelogenic polysaccharide into an aqueous solution containing gel-forming cations, through a syringe needle or another hollow feeder (nozzle) with an appropriate internal diameter, for instance of about 0.25 mm, more particularly, following the method of Smidsrød 0. and Skjåk-Bræk G. disclosed in TIBTECH-MARCH 1990, Vol. 8, p. 71-78, herein incorporated by reference, according to which the size of the droplets and of the gel beads thus obtained is controlled within the range desired for the scope of the present invention by means of an air stream coaxial with respect to the syringe inner diameter. The gel microspheres formed by contact with the water solution of gel-forming cations are maintained under stirring in the aforesaid solution for about 30 min to allow said cations to penetrate into the gel microspheres, are separated from the aqueous phase, e.g. by Gooch filtration, and dried.

According to another embodiment of the present process, which is particularly fit for commercial-scale application and does not require special feeders of the solutions, the gel matrix of the formulations of the present invention is obtained in the form of microgranules by pouring under vigorous stirring a homogeneous aqueous suspension of diacerhein and polysaccharide into a solution containing gel-forming cations (pouring being effects by means of a common feeder for laboratory or industrial scale, with no aid of the aforementioned syringe needle and coaxial air stream); separating the resulting gel particles agglomerates; drying and converting the agglomerates into microgranules by grinding, then sieving to separate the fractions with the selected particle size.

In particular, the gel particles agglomerates formed by contact with the water solution of gel-forming cations are kept under stirring for about 30 min before separation from the liquid phase excess, and grinding may be carried out, e.g., in a ball mill or in another apparatus suitable for grinding powders for pharmaceutical use. The microgranules obtained are sieved through convenient mesh sieve screens, with separation of the fractions having the desired particle size.

The gel matrix of the present invention, obtained according to the process variants illustrated above, contains diacerhein uniformly dispersed throughout, being thus different from the preparations in which microgranules including the active ingredient are coated with films of various types.

The aqueous suspension containing diacerhein and polysaccharide is typically a suspension in water and is typically obtained by addition of solid diacerhein to a solution, typically in water, containing at least an anionic polysaccharide, at a concentration typically ranging from 0.5% to 10% by weight, and in an amount corresponding to the solution-diacerhein ratio by weight specified above. The resulting mixture is then maintained under stirring until obtaining a homogeneous dispersion.

The water solution containing gel-forming cations is typically a solution in water and preferably comprises a water soluble salt of said cations, such as CaCl₂, at a concentration of said cations typically ranging from 5.10⁻⁴ M to 5 M, more preferably from 10⁻³ M to 1 M.

The quantity of the aforesaid cations, typically Ca⁺⁺ ions, added in respect of the anionic polysaccharide, may vary within wide limits, typically from about 0.01 moles to a shoichiometric excess in respect of the polysaccharide anionic groups, e.g. up to 10, and typically up to about 2 moles per mole of anionic groups, defined as the quantity of anionic groups neutralized by one mole of H⁺ ions.

H⁺/bivalent cation mixed hydrogels, in which the polysaccharide anionic groups are partially salified with said cations and are partially in the H⁺ form, are obtained from a solution of the cations used for gel formation also containing a protic acid, e.g. HCl.

According to a typical embodiment of the present invention, the water solution comprising the cations used for gel formation contains gel-forming cations in a stoichiometric defect in respect of the polymer anionic groups, e.g. from about 0.005 to about 0.05 mole of cations per mole of anionic groups, and further contains a protic acid, e.g. HCl, in a quantity preferably ranging from 0.5 to 5 moles, and typically from 0.5 to 1.2 moles of H⁺ per mole of polymer anionic groups.

Gel formation, in particular by addition of cations to anionic polysaccharides, typically takes place at room temperature, generally ranging from about +20°C to about +30°C.

Gel drying is typically conducted at atmospheric pressure, at temperatures ranging from +40°C to +100°C and more typically from +50°C to +80°C, the granular mass being typically dried in fluid bed apparatus.

Some particular embodiments of the invention are reported below, by way of indication and not of limitation.

### EXAMPLE 1 - Formulation of diacerhein in calcium alginate microspheres (D1)

Alginate PRONOVA MV (20 g) was dissolved in Milli-Q water (1 l). DAR (10 g) was suspended in the alginate solution by vigorous stirring. Once the suspension had become homogeneous, it was added dropwise through a syringe needle (internal diameter 0.25 mm) to 0.1 M CaCl₂ (2 l), according to the method disclosed in Smidsrød, O. and Skjåk-Bræk G., TIBTECH 8 MARCH (1990), 71-78. The gel microspheres formed by contact with the calcium solution were maintained under stirring in the CaCl₂ solution for about 30 min. The compound was recovered by Gooch filtration and dried on a fluid bed. The diacerhein content determined by the spectrophotometric method described below was 24% by weight.

### DIACERHEIN SPECTROPHOTOMETRIC DETERMINATION IN FORMULATIONS

Aliquots of each preparation (about 75 mg) were accurately weighed in 250 ml volumetric flasks. About 200 ml of phosphate buffer (0.1 M, pH 6.8) was added thereto. Each flask was maintained at room temperature for 48 hrs to complete dissolution of the compound. The resulting solution was made up to volume with phosphate buffer. Dilutions of each solution were prepared so as to obtain absorbance values ranging from 0.3 to 0.5 absorbance units. Readings were made by double-beam spectrophotometer using phosphate buffer as reference solution and determining the absorbance at 432 nm. A calibration curve was plotted with a mother liquor prepared by accurately weighing 25 mg diacerhein in a 250 ml flask and making up to volume with phosphate buffer (0.1 M, pH 6.8). A series of solutions at diacerhein concentration from 0.005 to 0.1 mg/ml was prepared by dilution. On the basis of the absorbance readout at 432 nm, a calibration curve was plotted to determine the diacerhein content in the aforesaid unknown-concentration solutions.

### EXAMPLE 2 - Formulation of diacerhein in H⁺/Ca⁺⁺ alginate mixed gel (D2)

Alginate PRONOVA MV (20 g) was dissolved in Milli-Q water (1 l). DAR (10 g) was suspended in the alginate solution by vigorous stirring. Once the suspension had become homogeneous, it was added dropwise through a syringe needle (internal diameter 0.25 mm) to 0.1 N HCl (2 l) containing 1 mM CaCl₂. The gel microspheres formed by contact with the calcium-containing acid solution were kept under stirring for about 30 min and then removed from the solution. The compound was recovered by Gooch filtration and dried on a fluid bed. The diacerhein content determined by the spectrophotometric method described above was 27% by weight.

### EXAMPLE 3 - Formulation of diacerhein in calcium polygalacturonate microspheres (D3)

Polygalacturonate acid (20 g) from apples (Sigma) was dissolved in Milli-Q water (250 ml). DAR (10 g) was suspended in the polygalacturonate acid solution by vigorous stirring. Once the suspension had become homogeneous, it was added dropwise through a syringe needle (internal diameter 0.25 mm) to 0.1 M CaCl₂ (500 ml).

The gel microspheres formed by contact with the calcium solution were maintained under stirring in CaCl₂ solution for about 30 min. The compound was recovered by Gooch filtration and dried on a fluid bed. (The microspheres were not particularly rigid and tended to lose their spherical shape probably due to the low molecular weight of polygalaturonic acid). The diacerhein content determined by the spectrophotometric method described above was 23% by weight.

### EXAMPLE 4 - Formulation of diacerhein in alginate/pectin mixed gels (D4)

Microgranules of diacerhein in alginate/pectin mixed gel were prepared as described in Example 1, but with the alginate (20 g) solution in water replaced by a solution of 10 g alginate PRONOVA MV and 10 g pectin from lemon peel in Milli-Q water (1 l). Gel microspheres were maintained under stirring for about 30 min, recovered, dried and analysed as described in Example 1.

The diacerhein content was 25% by weight.

### EXAMPLE 5 - Formulation of diacerhein in alginate/pectin mixed gels (D5)

Microgranules of diacerhein in alginate/pectin mixed gel were prepared as described in Example 1, but with the alginate (20 g) solution in water replaced by a solution of 10 g alginate PRONOVA MV and 20 g pectin from lemon peel in Milli-Q water (1 l) and with 10 g DAR replaced by 15 g DAR. The preparation was Gooch filtered, dried on a fluid bed, mixed with an equal amount of D(+)lactose, and ground.

The diacerhein content determined by the spectrophotometric method described above was 15% by weight.

### EXAMPLE 6 - Formulation of diacerhein in alginate/pectin mixed gels (D6)

Microgranules of diacerhein in alginate/pectin mixed gel were prepared as described in Example 1, but with the alginate (20 g) solution in water replaced by a solution of 10 g of alginate PRONOVA MV and 10 g of highly esterified pectin from apples in Milli-Q water (1 l). DAR (10 g) was suspended in the solution and once the suspension had become homogeneous, it was added dropwise through a syringe needle (internal diameter 0.25 mm) to 1 l, instead of 2 1, of 0.1 M CaCl₂. The gel microspheres were maintained under stirring for about 30 min, recovered, dried and analysed as described in Example 1.

The diacerhein content was 28% by weight.

### EXAMPLE 7 - Formulation of diacerhein in alginate gel (D7)

Microgranules of diacerhein encapsulated in alginate gel were prepared as described in Example 1, but with alginate water solution (20 g) replaced by a solution of 10 g of alginate PRONOVA MV dissolved in 1 l of lactose solution at 1% in Milli-Q water. DAR (10 g) was suspended in the solution and once the suspension had become homogeneous, it was added dropwise through a syringe needle (internal diameter 0.25 mm) to 0.1 M CaCl₂ (2 l) containing 1% lactose. The gel microspheres were maintained under stirring for about 30 min, recovered, dried and analysed as described in Example 1. The diacerhein content was 25% by weight.

### EXAMPLE 8 - Formulation containing diacerhein in alginate (D8)

Alginate PRONOVA MV (20 g) was dissolved in Milli-Q water (1 l). DAR (10 g) was suspended in the alginate solution by vigorous stirring. Once the suspension had become homogeneous, it was poured into 0.1 M CaCl₂ (2 l) maintained under stirring by a magnetic anchor. The gel agglomerates that formed by contact with the calcium solution were stirred for about 30 min in CaCl₂ solution to allow calcium ion diffusion also inside the aggregates. The compound was recovered by Gooch filtration and dried on a fluid bed. The dry preparation was ground and sieved through a 250 µm mesh sieve screen. The fraction with particle size below 250 µm was used for tolerability and bioavailability tests. The diacerhein content determined by the spectrophotometric method described above was 22% by weight.

### EXAMPLE 9 - Formulation containing diacerhein and pectin (D9)

HM pectin (10 g) was dissolved in Milli-Q water (500 mL). DAR (10 g) was suspended in the pectin solution by vigorous stirring. Once the suspension had become homogeneous, it was poured into 0.1 M CaCl₂ (2 l) maintained under stirring by a magnetic anchor. Pectin formed a gel by contact with the calcium solution. The system was stirred for about 30 min in CaCl₂ solution to allow gel stabilization with calcium diffusion also inside the aggregates. The compound was recovered by centrifugation and dried on a fluid bed. The dry preparation was ground and sieved through a 250 µm mesh sieve screen. The fraction with particle size below 250 µm was used for tolerability and bioavailability tests. The diacerhein content determined by the spectrophotometric method described above was 32% by weight.

### EXAMPLE 10 - Formulation containing diacerhein and pectin (D10)

HM pectin (50 g) was dissolved in Milli-Q water (1000 ml) in a mixer. DAR (25 g) was added and suspended in the pectin solution by vigorous stirring. Once the suspension had become homogeneous, it was added with 0.1 M CaCl₂ (500 ml) and the system was stirred for about 30 min. The gel formed was recovered and water excess was removed by filter press. The product was dried on a fluid bed. The dry product was ground in a ball mill to the desired particle size.

The diacerhein content determined by the spectrophotometric method described above was 31% by weight.

### COMPARATIVE TESTS

The formulations according to the present invention were compared with two commercial pharmaceutical preparations according to the known art, i.e. Fisiodar ^{R} and ART 50 ^{R}.

Fisiodar ^{R}, which is marketed in Italy, is a preparation in the form of gelatin capsules for oral administration, containing diacerhein, lactose and magnesium stearate.

Another pharmaceutical preparation based on diacerhein, known as ART 50 ^{R}, which is marketed in France, contains the same ingredients, i.e. diacerhein, lactose and magnesium stearate, as Fisiodar ^{R}, but in different amounts.

### GASTROINTESTINAL TOLERABILITY TESTS

Gastrointestinal tolerability tests on the new diacerhein formulations were conducted on the rat as test model. Male Hsd Brl:WH rats (basal weight: 250-300 g) were used. The parameters used for evaluating the gastrointestinal tolerability of the various formulations were animal weight gain, occurrence and severity of adverse reactions, in particular diarrhea.

Four groups (each of 10 animals) received an oral dose of 80 mg/kg/die diacerhein for 14 consecutive days, by administration of ART 50 R (commercial formulation) and, respectively, of the preparations of the present invention, D6, D8 and D9. ART 50 ^{R} was administered as a suspension in 10% gum arabic while formulations D6, D8 and D9 were mixed with condensed milk. A control group of 5 animals (5th group) was treated according to the above process with 10% gum arabic solution.

### Weight gain

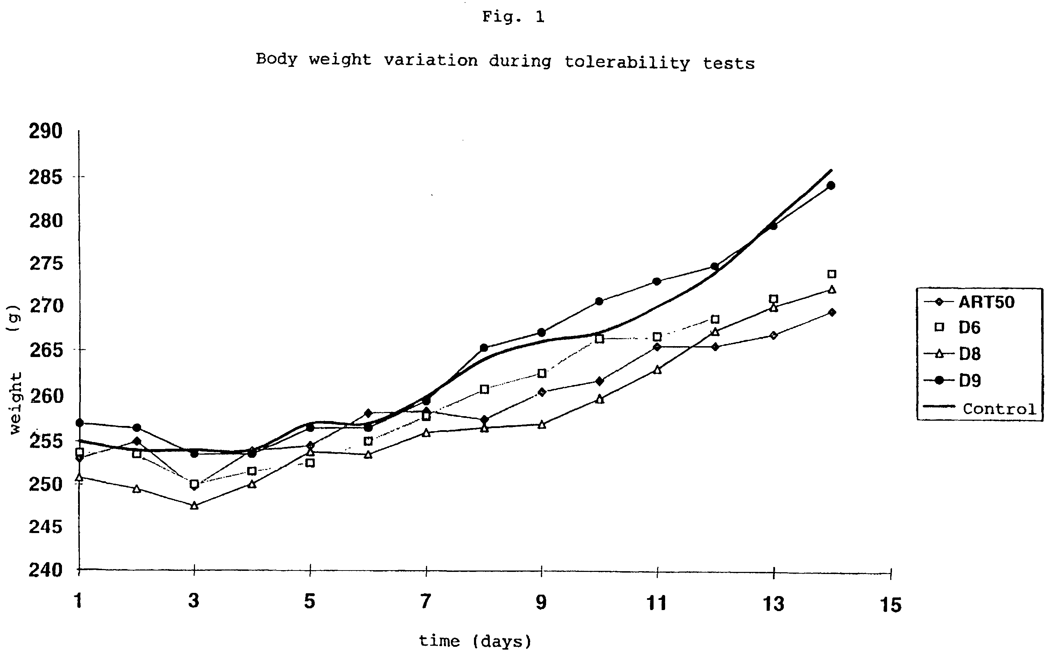

The results concerning the average weight gain in the various groups are reported in Table 1 and Figure 1. As concerns unpaired data, the statistical analysis was performed by Student's t-test.

**Table 1 -**

| **Body weights (BW) analysis** | | | | | |
|---|---|---|---|---|---|
| | Control | ART 50 | D6 | D8 | D9 |
| W^{1st}(average wt., 1st day) | 255g | 253g | 254g | 251g | 257g |
| W^{14th}(average wt., 14th day) | 286g | 270g | 274g | 272g | 284g |
| _ W=W^{14th}-W^{1st} | 31 | 17 | 20 | 21 | 27 |
| S.D. | 7 | 10 | 8 | 10 | 3 |
| W^{1st} = average weight, 1st day W^{14th} = average weight, 14th day S.D. = Standard Deviation | | | | | |

The analysis of the results shows that:
a) at the end of the treatment, the control group showed a weight gain of 31 ± 7 g (x ± S.D.);
b) the groups treated with the various diacerhein formulations showed a lower weight gain when compared with the control group.
   The lowest weight gain was observed in the group treated with ART 50: control (31 ± 7 g) > D9 (27 ± 3 g); > D8 (21 ± 10 g); > D6 (20 ± 8 g); > ART 50 (17 ± 10 g);
c) as concerns the parameter "weight gain", all three new formulations, D9, D8 and D6, were found to be better tolerated than ART 50, with a % weight gain that, at the end of treatment, was higher than that secured by ART 50:
   D9 (+10.6%) > D8 (+8.5%) > D6 (+8%) > ART 50 (+6%).

The weight difference between the groups treated with D6, D8 and D9 and the groups treated with ART 50 shows that the compositions of the invention are definitely better than ART 50 as concerns gastrointestinal tolerability, D9 giving the best results.

### Diarrhea episodes

As concerns the second parameter for the evaluation of gastrointestinal tolerability, the number and severity of diarrhea episodes in the groups treated with ART 50, D6, D8 and D9 and in the control group were determined.

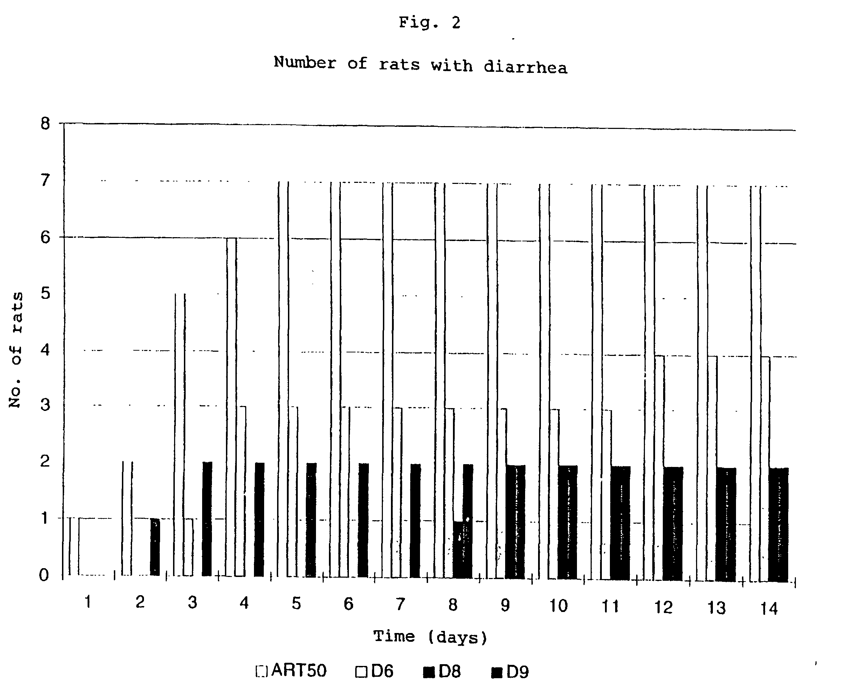

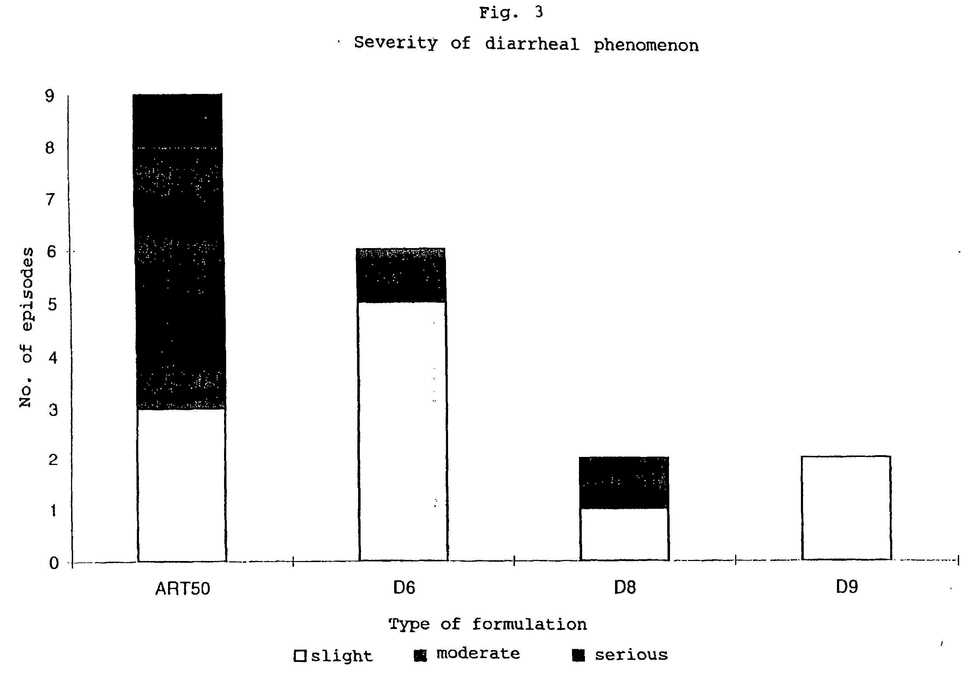

The incidence of diarrheal phenomena, expressed as the number of rats with at least one diarrhea episode and the severity of same - severity being meant as the number of diarrhea episodes occurred in the various test groups - are reported in Figs. 2 and 3 respectively. It can be observed that:
a) No animal of the control group showed adverse reactions and in particular diarrhea.
b) Seven out of 10 animals of the group treated with ART 50 suffered from diarrhea episodes (for a total of 9 episodes); diarrhea was observed in 4 out of 10 animals of the group treated with D6 (6 episodes) and in 2 out of 10 animals of the groups treated with D8 and D9.
c) Diarrhea was averagely more severe in the group treated with ART 50 than in the other treated groups.

### Conclusions

The conclusion can be drawn that the new formulations, when compared with commercial ART 50, exhibit a higher gastrointestinal tolerability as regards the average weight gain and the occurrence of diarrhea episodes. Therefore, the new formulations minimize the number of diarrhea episodes occurring during the treatment of osteoarthrosis.

### BIOAVAILABILITY TESTS VERSUS A 40 mg/kg SINGLE DOSE

Gastrointestinal absorption of ART 50 and of some new preparations was evaluated on groups of 3 animals, each weighing between 250 and 300 g housed separately in metabolic cages and fed in a normal way.

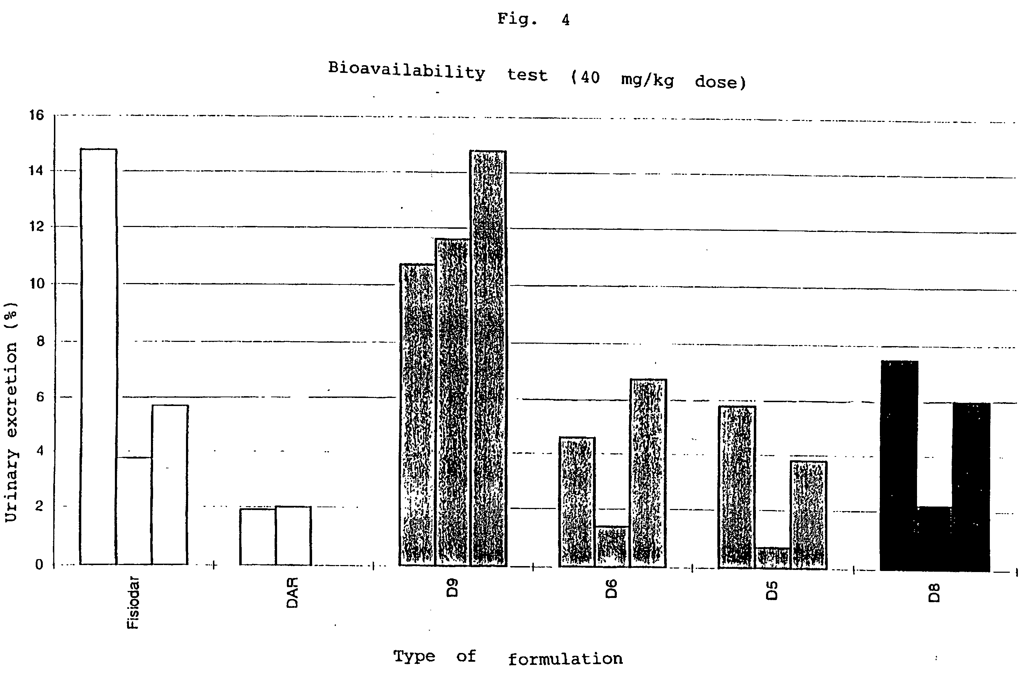

Each rat received a single oral dose of preparation corresponding to 40 mg/kg of diacerhein. The 24 hours urine was collected and assayed by the HPLC method described hereinafter to determine the content of diacerhein and metabolites thereof. The diacerhein % amount, evaluated as urinary excretion of rhein in each test group, is reported in Fig. 4.

### BIOAVAILABILITY TESTS VERSUS A 2 mg/kg SINGLE DOSE

Gastrointestinal absorption of ART 50 and of new preparations D8 and D9 was evaluated on groups of 3 animals weighing between 250 and 300 g each housed separately in metabolic cages and kept without food. Each rat received a single oral dose of preparation corresponding to 2 mg/kg diacerhein. The 24 hours urine was collected and assayed by the HPLC method described hereinafter to determined the content of diacerhein and metabolites thereof. The diacerhein % amount, evaluated as urinary excretion of rhein in each group, is reported in Fig. 5.

### METHOD FOR THE DETERMINATION OF DIACERHEIN AND METABOLITES THEREOF IN BIOLOGICAL SAMPLES

The urinary excretion of diacerhein was determined according to the reserved-phase HPLC method described in Springolo, V. and Coppi, G., *J. of Chromatography*, **428,** 1988, 173-177. Urine samples were treated with β-glucoronidase from Helix pomataia (Sigma, type H₂) to hydrolyse the metabolites (glucoronide and rhein sulphate) present in the biological liquid as described in Magnard, O., Louchahi, K., *et al., Biopharmaceutics and Drug Disposition*, **14,** 1993, 401-408, and Debord, P., Louchahi, K., *et al*., *Fund. Clin. Pharmacol*., **7**, 1993, 435-441.

An enzymatic solution (100 µl; 500 IU/ml), 40 µl of acetate buffer (pH 4.9), and 360 µl of water were added to 500 µl of urine and incubated at 37°C for 2 hrs. Urine samples were then treated with an equal volume of acetonitrile. The mixture was Vortex agitated for 1 min and centrifuged at 5000 rpm for 20 min. The supernatant was transferred to a test tube and added with a proper volume of McIlvaine (pH 2.2)-acetonitrile buffer (55:45). The sample solution (20 µl) was injected into a reserved-phase column (Alltima RP-8). The eluent was a McIlvaine-acetonitrile buffer mixture (55:45) as described in Springolo, V. and Coppi, G., *J*. *of Chromatography*, **428,** 1988, 173-177.

The wave length of UV-visible detector was set at 258 nm for a higher sensitivity of the method. The calibration curve was obtained by addition of known amounts of rhein to urine samples of drug-untreated rats. The standard reference solutions were subjected to the same tests as described for urine samples of rats treated with the various formulations. Fig. 6 shows the calibration curve for the determination of the diacerhein content evaluated as rhein in biological fluids.

## Claims

1. Formulation containing diacerhein, **characterized in that** diacerhein is incorporated into a hydrogel matrix containing at least a ionic polysaccharide gelled by addition of electrolytes.

2. Formulation according to claim 1, further comprising pharmaceutically acceptable excipients or diluents or mixtures thereof.

3. The formulation according to any preceding claims, in the form of microgranules.

4. The formulation according to claim 3, wherein the microgranules particle size ranges between 5 and 500 µm.

5. The formulation according to claim 4, wherein the microgranules particle size ranges between 25 and 350 µm.

6. The formulation according to claim 5, wherein the microgranules particle size ranges between 50 and 100 µm.

7. The formulation according to claim 1, wherein the hydrogel matrix comprises at least an anionic polysaccharide at least partially salified with gel-forming cations.

8. The formulation according to claim 7, wherein the anionic polysaccharide is selected from a polysaccharide containing carboxyl groups and a polysaccharide containing sulphate groups.

9. The formulation according to claim 7, wherein the polysaccharide containing carboxyl groups is selected from the group consisting of pectins, alginates and polygalacturonans, and the polysaccharide containing sulphate groups is a carrageenan.

10. The formulation according to claim 7 or claim 9, wherein the polysaccharide contains carboxyl groups.

11. The formulation according to claim 7, wherein the polysaccharide is selected from the group consisting of alginates, pectins and mixtures thereof.

12. The formulation according to claim 11, wherein the polysaccharide is a pectin.

13. The formulation according to claim 12, wherein the pectin methylation degree is above 50%.

14. The formulation according to claim 12, wherein the pectin methylation degree ranges from 70% to 95%.

15. The formulation according to claim 7, wherein the cations are selected from alkaline earth metal cations and transition metal cations.

16. The formulation according to claim 7, wherein the cations are selected from the group consisting of Ca⁺⁺, Al⁺⁺⁺, and Cu⁺⁺.

17. The formulation according to claim 7, wherein the cations are Ca⁺⁺ ions.

18. The formulation according to claim 7, wherein the polysaccharide is an anionic polysaccharide selected from the group consisting of pectins, alginates and mixtures thereof, and the cations are Ca⁺⁺ ions.

19. The formulation according to claim 7, wherein the polysaccharide is a pectin and the cations are Ca⁺⁺ ions.

20. The formulation according to claim 7, wherein the anionic groups of the said polysaccharide are carboxyl groups partially salified with Ca⁺⁺ ions and partially in the H⁺ form.

21. The formulation according to claim 1, wherein the polymer/diacerhein ratio ranges from 0.1:1 to 10:1 by weight.

22. The formulation according to claim 21, wherein the said ratio ranges from 1:1 to 3:1.

23. The formulation according to claims from 1 through 22 containing diacerhein (from 10% to 50% by weight), anionic polysaccharide (from 10% to 80% by weight), water (from 5% to 20% by weight), gel-forming cations (from 1% to 30% by weight).

24. The formulation according to claim 23, further containing lactose (from 10% to 80% by weight).

25. Process for the preparation of a formulation according to any preceding claim, comprising gelling a mixture of diacerhein and of an ionic polysaccharide in an aqueous diluent by addition of electrolytes, separating the gel from the liquid phase excess, and drying the gel.

26. The process according to claim 25, wherein the polysaccharide is an anionic polysaccharide and gelling is obtained by addition of gel-forming cations.

27. The process according to claim 26, wherein the polysaccharide subjected to gelling is in the form of a water soluble salt and the gel-forming cations are added in the form of water soluble salts.

28. The process according to claim 26, comprising pouring under stirring an aqueous suspension containing diacerhein and the selected polysaccharide into an aqueous solution containing gel-Forming cations, keeping under stirring, separating the gel from the aqueous phase, and drying the gel.

29. The process according to claim 28, wherein the gel formed by contact with the aqueous solution containing the gel-forming cations is kept under stirring from 15 min to 2 hours.

30. The process according to claim 29, wherein the said contact lasts about 30 min.

31. The process according to claim 29, wherein the gel is directly obtained in the form of microspheres by dropping an aqueous suspension containing diacerhein and the gelogenic polysaccharide selected into an aqueous solution containing gel-forming cations, through a syringe needle or another hollow feeder (nozzle).

32. The process according to claim 29 suitable for obtaining gel in the form of microgranules, comprising the steps of pouring, under vigorous stirring, a homogeneous aqueous suspension of diacerhein and polysaccharide into a solution containing gel-forming cations, separating the resulting gel particles agglomerates, drying and converting same into microgranules by grinding.

33. The process according to claim 29, wherein the aqueous suspension containing diacerhein and polysaccharide is obtained by addition of solid diacerhein to a water solution containing at least an anionic polysaccharide, at a concentration ranging from 0.5% to 10% by weight and in a weight ratio in respect of diacerhein ranging from 0.1:1 to 10:1.

34. The process according to claim from 29 through 33, wherein the polysaccharide is selected from the group consisting of alginates, pectins and mixtures thereof, and the cations are Ca⁺⁺ ions.

35. The process according to claim 34, wherein Ca⁺⁺ ions are added as an aqueous solution of a Ca⁺⁺ soluble salt, having a gel-forming cations concentration ranging from 5.10⁻⁴ M to 5 M and a quantity of gel-forming cations ranging from 0.01 to 10 moles per mole of polysaccharide anionic groups.

36. The process according to claim 35, wherein the concentration of gel-forming cations ranges from 10⁻³ M to 1 M and the amount of same ranges from 0.01 to 2 moles per mole of polysaccharide anionic groups.

37. The process according to claim 29 for the preparation of H⁺/gel-forming cation mixed hydrogels, in which the gel-forming cations aqueous solution contains gel-forming cations in a stoichiometric defect in respect of the polysaccharide anionic groups, and furthermore contains a protic acid.

38. The process according to claim 37, wherein the amount of gel-forming cations ranges from 0.005 to 0.5 mole per mole of anionic cations and the amount of acid ranges from 0.5 to 5 moles per mole of anionic groups.

39. The process according to claim 29, wherein gel formation is carried out at room temperature.

40. The process according to claim 29, wherein gel drying takes place at atmospheric pressure and at a temperature ranging from 40°C to 100°C.

41. Diacerhein-containing formulation obtainable according to any of the preceding claims from 25 through 40.

42. Diacerhein-containing pharmaceutical compositions comprising at least a formulation according to any of the preceding claims from 1 to 24 or according to claim 41, optionally further containing pharmaceutically acceptable excipients or diluents or mixtures thereof.

43. The pharmaceutical compositions according to claim 42 suitable for oral administration.

44. Use of a formulation according to any of the preceding claims from 1 to 24 or according to claim 41 for the preparation of pharmaceutical compositions useful as analgesic and antiinflammatory compositions.

## Patentansprüche

1. Diacerhein enthaltende Rezeptur, **dadurch gekennzeichnet, dass** Diacerhein Bestandteil einer Hydrogelmatrix ist, die mindestens ein ionisches Polysaccharid enthält, das durch Hinzufügung von Elektrolyten in ein Gel übergeführt wurde.

2. Rezeptur gemäß Anspruch 1, **dadurch gekennzeichnet, dass** sie darüber hinaus pharmazeutisch annehmbare Arzneiträgerstoffe, Verdünnungsmittel oder eine Mischung daraus enthält.

3. Rezeptur gemäß einem der vorstehenden Ansprüche, **dadurch gekennzeichnet, dass** sie in Form von Mikrogranulat vorliegt.

4. Rezeptur gemäß Anspruch 3, **dadurch gekennzeichnet, dass** die Körnchengröße des Mikrogranulats zwischen 5 und 500 µm liegt.

5. Rezeptur gemäß Anspruch 4, **dadurch gekennzeichnet, dass** die Körnchengröße des Mikrogranulats zwischen 25 und 350 µm liegt.

6. Rezeptur gemäß Anspruch 5, **dadurch gekennzeichnet, dass** die Körnchengröße des Mikrogranulats zwischen 50 und 100 µm liegt.

7. Rezeptur gemäß Anspruch 1, **dadurch gekennzeichnet, dass** die Hydrogelmatrix mindestens ein anionisches Polysaccharid enthält, das zumindest teilweise durch Gel bildende Kationen zu Salz umgesetzt wurde.

8. Rezeptur gemäß Anspruch 2, **dadurch gekennzeichnet, dass** das anionische Polysaccharid aus einem Polysaccharid mit Karboxylgruppen und einem Polysaccharid mit Sulfatgruppen ausgewählt wird.

9. Rezeptur gemäß Anspruch 7, **dadurch gekennzeichnet, dass** das Polysaccharid mit Karboxylgruppen aus einer Gruppe bestehend aus Pektinen, Alginaten und Polygalakturonanen ausgewählt wird und es sich beim Polysaccharid mit Sulfatgruppen um ein Carragheenan handelt.

10. Rezeptur gemäß Anspruch 7 oder Anspruch 9, **dadurch gekennzeichnet, dass** das Polysaccharid Karboxylgruppen enthält.

11. Rezeptur gemäß Anspruch 7, **dadurch gekennzeichnet, dass** das Polysaccharid aus einer Gruppe ausgewählt wird, die Alginate, Pektine und diesbezügliche Mischungen enthält.

12. Rezeptur gemäß Anspruch 11, **dadurch gekennzeichnet, dass** das Polysaccharid ein Pektin ist.

13. Rezeptur gemäß Anspruch 12, **dadurch gekennzeichnet, dass** der Methylierungsgrad für Pektin über 50% liegt.

14. Rezeptur gemäß Anspruch 12, **dadurch gekennzeichnet, dass** der Methylierungsgrad für Pektin zwischen 70% und 95% liegt.

15. Rezeptur gemäß Anspruch 7, **dadurch gekennzeichnet, dass** die Kationen unter denen der Erdalkalimetalle und der Übergangsmetalle ausgewählt werden.

16. Rezeptur gemäß Anspruch 7, **dadurch gekennzeichnet, dass** die Kationen ausgewählt werden aus der Gruppe, die aus Ca⁺⁺, Al⁺⁺⁺ und Cu⁺⁺ besteht.

17. Rezeptur gemäß Anspruch 7, **dadurch gekennzeichnet, dass** die Kationen aus Ca⁺⁺-lonen bestehen.

18. Rezeptur gemäß Anspruch 7, **dadurch gekennzeichnet, dass** das Polysaccharid ein anionisches Polysaccharid ist, das aus der Gruppe mit Pektinen, Alginaten und diesbezüglichen Mischungen besteht und die Kationen Ca⁺⁺-lonen sind.

19. Rezeptur gemäß Anspruch 7, **dadurch gekennzeichnet, dass** das Polysaccharid ein Pektin ist und die Kationen Ca⁺⁺-lonen sind.

20. Rezeptur gemäß Anspruch 7, **dadurch gekennzeichnet, dass** die anionischen Gruppen des genannten Polysaccharids Karboxylgruppen sind, die mit Ca⁺⁺-lonen zu Salzen umgesetzt sind und teilweise in H⁺-Form vorliegen.

21. Rezeptur gemäß Anspruch 1, **dadurch gekennzeichnet, dass** das Polymer/Diacerhein-Verhältnis gewichtsmäßig zwischen 0,1:1 und 10:1 liegt.

22. Rezeptur gemäß Anspruch 21, **dadurch gekennzeichnet, dass** das vorgenannten Verhältnis zwischen 1:1 und 3:1 liegt.

23. Rezeptur gemäß Anspruch 1 bis einschließlich 22, **dadurch gekennzeichnet, dass** sie Diacerhein (mit 10 bis 50 Gewichtsprozent), ein anionisches Polysaccharid (mit 10 bis 80 Gewichtsprozent), Wasser (mit 5 bis 20 Gewichtsprozent) und Gel bildende Kationen (mit 1 bis 30 Gewichtsprozent) enthält.

24. Rezeptur gemäß Anspruch 23, **dadurch gekennzeichnet, dass** sie darüber hinaus Laktose (von 10 bis 80 Gewichtsprozent) enthält.

25. Verfahren für die Zubereitung einer Rezeptur gemäß einem der vorstehenden Ansprüche, **dadurch gekennzeichnet, dass** eine Mischung aus Diacerhein und einem ionischen Polysaccharid in einem wässrigen Verdünnungsmittel durch Hinzufügung von Elektrolyten in ein Gel übergeführt, das Gel von der überschüssigen Flüssigphase getrennt und getrocknet wird.

26. Verfahren gemäß Anspruch 25, **dadurch gekennzeichnet, dass** das Polysaccharid anionisch ist und die Gelbildung durch Hinzufügung Gel bildender Kationen erfolgt.

27. Verfahren gemäß Anspruch 26, **dadurch gekennzeichnet, dass** das der Gelbildung unterworfene Polysaccharid in Form eines wasserlöslichen Salzes vorliegt und die Gel bildenden Kationen in Form von wasserlöslichen Salzen hinzugefügt werden.

28. Verfahren gemäß Anspruch 26, **dadurch gekennzeichnet, dass** eine wässrige Suspension, die Diacerhein und das ausgewählte Polysaccharid enthält, unter ständigem Umrühren in eine wässrige Lösung mit Gel bildenden Kationen geschüttet, der Rührvorgang weitergeführt, das Gel von der wässrigen Phase getrennt und getrocknet wird.

29. Verfahren gemäß Anspruch 28, **dadurch gekennzeichnet, dass** das Gel, welches im Kontakt mit jener wässrigen Lösung gebildet wurde, die Gel bildende Kationen enthält, zwischen 15 Minuten und 2 Stunden weiter gerührt wird.

30. Verfahren gemäß Anspruch 29, **dadurch gekennzeichnet, dass** der genannte Kontakt etwa 30 Minuten dauert.

31. Verfahren gemäß Anspruch 29, **dadurch gekennzeichnet, dass** das Gel unmittelbar in Form von Mikrokügelchen in der Weise erhalten wird, dass eine wässrige Suspension mit Diacerhein und dem gewählten Gel bildenden Polysaccharid in eine wässrige Lösung eingebracht wird, welche Gel bildende Kationen enthält, wofür eine Injektionsnadel oder ein anderes hohles Zuführelement (Düse) verwendet wird.

32. Verfahren gemäß Anspruch 29, **dadurch gekennzeichnet, dass** es sich zur Herstellung von Gelen in Form von Mikrokügelchen eignet, bestehend aus den Verfahrensschritten des Zuschüttens unter kräftigem Umrühren einer homogenen wässrigen Suspension mit Diacerhein und dem Polysaccharid in eine Lösung mit Gel bildenden Kationen, die Abtrennung der sich ergebenden Ansammlung aus Gelpartikeln, deren Trocknung und Umwandlung in Mikrokügelchen durch einen Schleifvorgang umfasst.

33. Verfahren gemäß Anspruch 29, **dadurch gekennzeichnet, dass** die wässrige Lösung mit Diacerhein und Polysaccharid durch Hinzufügen von Diacerhein in fester Form zu einer wässrigen Lösung gewonnen wird, welche mindestens ein anionisches Polysaccharid in Konzentrationswerten von 0,5 bis 10 Gewichtsprozent und einem Gewichtsverhältnis hinsichtlich des Diacerheins von 0,1:1 bis 10:1 enthält.

34. Verfahren gemäß Anspruch 29 bis einschließlich 33, **dadurch gekennzeichnet, dass** das Polysaccharid aus der Gruppe mit Alginaten, Pektinen und diesbezüglichen Mischungen gewählt wird, und dass die Kationen Ca⁺⁺-lonen sind.

35. Verfahren gemäß Anspruch 34, **dadurch gekennzeichnet, dass** Ca⁺⁺lonen als wässrige Lösung eines löslichen Ca⁺⁺-Salzes hinzugefügt werden, die eine Konzentration an Gel bildenden Kationen im Bereich von 5,10⁻⁴ M bis 5 M aufweist, wobei die Menge an Gel bildenden Kationen von 0,01 bis 10 Mol pro Mol der anionischen Gruppen im Polysaccharid reicht.

36. Verfahren gemäß Anspruch 35, **dadurch gekennzeichnet, dass** die Konzentration der Gel bildenden Kationen von 10⁻³ M bis 1 M reicht und die Menge dieser Verbindungen zwischen 0,01 und 2 Mol pro Mol an anionischen Gruppen des Polysaccharids liegt.

37. Verfahren gemäß Anspruch 29 für die Zubereitung von Mischhydrogelen mit H⁺/Gel bildenden Kationen, **dadurch gekennzeichnet, dass** die wässrige Lösung mit Gel bildenden Kationen diese Gel bildenden Kationen an einer stöchiometrischen Fehlstelle der anionischen Gruppen des Polysaccharids enthält und darüber hinaus eine Proteinsäure enthält.

38. Verfahren gemäß Anspruch 37, **dadurch gekennzeichnet, dass** die Menge der Gel bildenden Kationen zwischen 0,005 und 0,5 Mol pro Mol an anionischen Kationen liegt und die Säuremenge zwischen 0,5 und 5 Mol pro Mol an anionischen Gruppen liegt.

39. Verfahren gemäß Anspruch 29, **dadurch gekennzeichnet, dass** die Gelbildung bei Raumtemperatur stattfindet.

40. Verfahren gemäß Anspruch 29, **dadurch gekennzeichnet, dass** die Geltrocknung bei Atmosphärendruck und einer Temperatur zwischen 40°C und 100°C stattfindet.

41. Rezeptur mit Diacerheingehalt, **dadurch gekennzeichnet, dass** sie sich nach jedem beliebigen der vorstehenden Ansprüche zwischen 25 und einschließlich 40 gewinnen lässt.

42. Diacerheinhaltige pharmazeutische Zusammensetzungen, **dadurch gekennzeichnet, dass** sie mindestens eine Rezeptur nach einem beliebigen der vorstehenden Ansprüche von 1 bis 24 oder gemäß Anspruch 41 enthalten, und nach Wahl weitere pharmazeutisch annehmbare Arzneistoffträger, Verdünnungsmittel oder eine diesbezügliche Mischung enthalten können.

43. Die pharmazeutischen Präparate gemäß Anspruch 42, **dadurch gekennzeichnet, dass** sie geeignet für eine Verabreichung *per os* sind.

44. Verwendung einer Rezeptur nach einem beliebigen der vorstehenden Ansprüche von 1 bis 24 bzw. gemäß Anspruch 41, **dadurch gekennzeichnet, dass** sie für die Herstellung pharmazeutischer Präparate geeignet sind, die als schmerz- und entzündungshemmende Zubereitungen verwendet werden können.

## Revendications

1. Formulation contenant de la diacérhéine, **caractérisée en ce que** ladite diacérhéine est incorporée à une matrice hdrogel contenant au moins un polysaccharide ionique gélifié par addition d'électrolytes.

2. Formulation selon la revendication 1, comprenant aussi des excipients ou diluants ou mélanges de ceux-ci, pharmaceutiquement acceptables.

3. Formulation selon l'une quelconque des revendications précédentes, se présentant sous la forme de microgranulés.

4. Formulation selon la revendication 3, dans laquelle la taille particulaire des microgranulés est comprise entre 5 et 500 µm.

5. Formulation selon la revendication 4, dans laquelle 1a taille particulaire des microgranulés est comprise entre 25 et 350 µm.

6. Formulation selon la revendication 5, dans laquelle la taille particulaire des microgranulés est comprise entre 50 et 100 µm.

7. Formulation selon la revendication 1, dans laquelle la matrice hydrogel comprend au moins un polysaccharide anionique, salifié au moins partiellement par des cations formateurs de gel.

8. Formulation selon la revendication 7, dans laquelle le polysaccharide anionique est choisi à partir d'un polysaccharide contenant des groupes carboxyle et d'un polysaccharide contenant des groupes sulfate.

9. Formulation selon la revendication 7, dans laquelle le polysaccharide contenant des groupes carboxyle est choisi au sein du groupe comprenant les pectines, les alginates et les polygalacturonans, et le polysaccharide contenant des groupes sulfate est un carragheen.

10. Formulation selon la revendication 7 ou la revendication 9, dans laquelle le polysaccharide contient des groupes carboxyle.

11. Formulation selon la revendication 7, dans laquelle le polysaccharide est choisi au sein du groupe comprenant les alginates, les pectines et les mélanges de ceux-ci.

12. Formulation selon la revendication 11, dans laquelle la polysaccharide est une pectine.

13. Formulation selon la revendication 12, dans laquelle le degré de méthylation de la pectine est supérieur à 50 %.

14. Formulation selon la revendication 12, dans laquelle le degré de méthylation de la pectine est compris entre 70 % et 95 %.

15. Formulation selon la revendication 7, dans laquelle les cations sont choisis parmi les cations de métaux alcalinoterreux et les cations de métaux de transition.

16. Formulation selon la revendication 7, dans laquelle les cations sont choisis au sein du groupe comprenant Ca⁺⁺, Al⁺⁺⁺ et Cu⁺⁺.

17. Formulation selon la revendication 7, dans laquelle les cations sont des ions Ca⁺⁺.

18. Formulation selon la revendication 7, dans laquelle le polysaccharide est un polysaccharide anionique choisi au sein du groupe comprenant les pectines, les alginates et leurs mélanges, et les cations sont des ions Ca⁺⁺.

19. Formulation selon la revendication 7, dans laquelle le polysaccharide est une pectine, et les cations sont des ions Ca⁺⁺.

20. Formulation selon la revendication 7, dans laquelle les groupes anioniques dudit polysaccharide sont des groupes carboxyle partiellement salifiés par des ions Ca⁺⁺ et partiellement sous forme H⁺.

21. Formulation selon la revendication 1, dans laquelle le rapport pondéral polymère/diacérhéine est compris entre 0,1/1 et 10/1.

22. Formulation selon la revendication 21, dans laquelle ledit rapport est compris entre 1/1 et 3/1.

23. Formulation selon l'une quelconque des revendications 1 à 22, contenant de la diacérhéine (de 10 % à 50 % en poids), un polysaccharide anionique (de 10 % à 80 % en poids), de l'eau (de 5 % à 20 % en poids), des cations formateurs de gel (de 1 % à 30 % en poids).

24. Formulation selon la revendication 23, contenant également du lactose (de 10 % à 80 % en poids).

25. Procédé de préparation d'une formulation selon l'une quelconque des revendications précédentes, comprenant la gélification d'un mélange de diacérhéine et d'un polysaccharide ionique dans un diluant aqueux par addition d'électrolytes, la séparation du gel et de la phase aqueuse en excès, et le séchage du gel.

26. Procédé selon la revendication 25, dans lequel le' polysaccharide est un polysaccharide anionique, et dans lequel la gélification est obtenue par addition de cations formateurs de gel.

27. Procédé selon la revendication 26, dans lequel le polysaccharide soumis à la gélification est sous la forme d'un sel hydrosoluble, et dans lequel les cations formateurs de gel sont additionnés sous la forme de sels hydrosolubles.

28. Procédé selon la revendication 26, comprenant le coulage sous agitation, d'une suspension aqueuse contenant la diacérhéine et le polysaccharide choisi, dans une solution aqueuse contenant les cations formateurs de gel, le maintien de l'agitation, la séparation du gel et de la phase aqueuse, et le séchage du gel.

29. Procédé selon la revendication 28, dans lequel le gel formé par le contact avec la solution aqueuse contenant les cations formateurs de gel est maintenu sous agitation pendant 15 minutes à 2 heures.

30. Procédé selon la revendication 29, dans lequel ledit contact dure environ 30 minutes.

31. Procédé selon la revendication 29, dans lequel le gel est directement obtenu sous forme de microsphères en laissant goutter une suspension contenant la diacérhéine et le polysaccharide générateur de gel choisi, dans une solution aqueuse contenant des cations formateurs de gel, à travers l'aiguille d'une seringue ou par un autre système d'alimentation creux (buse).

32. Procédé selon la revendication 29, approprié à l'obtention d'un gel sous forme de microgranulés, comprenant les étapes de coulée sous forte agitation, d'une suspension aqueuse homogène de diacérhéine et de polysaccharide dans une solution contenant des cations formateurs de gel, de séparation des agglomérats des particules de gel obtenus, de séchage et de conversion de ceux-ci en microgranulés, par broyage.

33. Procédé selon la revendication 29, dans lequel la suspension aqueuse contenant la diacérhéine et le polysaccharide est obtenue par addition de ladite diacérhéine solide à une solution aqueuse contenant au moins un polysaccharide anionique à une concentration comprise entre 0,5 % et 10 % en poids, et dans un rapport pondéral par rapport à la diacérhéine, compris entre 0,1/1 et 10/1.

34. Procédé selon les revendications 29 à 33, dans lequel le polysaccharide est choisi au sein du groupe comprenant les alginates, les pectines et leurs mélanges, et les cations sont les ions Ca⁺⁺.

35. Procédé selon la revendication 34, dans lequel des ions Ca⁺⁺ sont ajoutés sous forme de solution aqueuse d'un sel de calcium hydrosoluble, ayant une concentration de cations formateurs de gel comprise entre 5,10⁻⁴M et 5M, et une quantité de cations formateurs de gel comprise entre 0,01 et 10 moles par mole de groupes anioniques de polysaccharide.

36. Procédé selon la revendication 35, dans lequel la concentration des cations formateurs de gel est comprise entre 10⁻³M et 1M et le quantité de ceux-ci est comprise entre 0,01 et 2 moles par mole de groupes anioniques de polysaccharide.

37. Procédé selon la revendication 29, pour la préparation d'hydrogels mixtes H⁺/cation formateur de gel, dans lequel la solution aqueuse de cations formateurs de gel contient des cations formateurs de gel en défaut stoechiométrique par rapport aux groupes anioniques du polysaccharide, et qui contient en plus, un acide protique.

38. Procédé selon la revendication 37, dans lequel la quantité de cations formateurs de gel est comprise entre 0,005 et 0,5 mole par mole de groupes anioniques et la quantité d'acide est comprise entre 0,5 et 5 moles par mole de groupes anioniques.

39. Procédé selon la revendication 29, dans lequel la formation de gel est effectuée à la température ambiante.

40. Procédé selon la revendication 29, dans lequel le séchage du gel a lieu à la pression atmosphérique et à une température comprise entre 40°C et 100°C.

41. Formulation contenant de la diacérhéine, pouvant être obtenue selon l'une quelconque des revendications précédentes 25 à 40.

42. Compositions pharmaceutiques contenant de la diacérhéine, comprenant au moins une formulation selon l'une quelconque des revendications précédentes 1 à 24, ou selon la revendication 41, contenant éventuellement aussi des excipients ou diluants ou mélanges de ceux-ci, pharmaceutiquement acceptables.

43. Compositions pharmaceutiques selon la revendication 42, appropriées à l'administration par voie orale.

44. Utilisation d'une formulation selon l'une quelconque des revendications précédentes 1 à 24, ou selon la revendication 41, pour la préparation de compositions pharmaceutiques utiles en tant que compositions analgésiques et anti-inflammatoire.
